# EUROPEAN PATENT APPLICATION

(11) **EP 4 844 777 A1**
(43) Date of publication of application: **25.11.2026**
(21) Application number: 26179539.7
(22) Date of filing: 19.05.2026
(51) Int. Cl.: G16H 20/00, G06Q 30/0601, G16H 70/40

(54) **METHOD AND SYSTEM FOR PROVIDING CUSTOMIZED SERVICES LINKED TO PRESCRIPTIONS**

(30) Priority: 20.05.2025 KR 20250065694
(71) Applicant: RBIZ INC., Paju-si, Gyeonggi-do 10908 (KR)
(72) Inventor: PAE, Heecheol, 06701 Seocho-gu, Seoul (KR)
(74) Representative: ABG Intellectual Property Law, S.L.

(57) **Abstract**

An electronic device and its operation method that provides customized services linked to prescriptions by portalizing application of AI green welfare card system based on collection and provision of user data, are disclosed. The electronic device according to the present disclosure includes a user interface configured to receive user input, a communication interface configured to perform communication connection with a server, a display configured to output visual content, and a processor connected to the user interface, the communication interface, and the display, and capable of performing control operations by executing at least one instruction, wherein the processor may identify prescription information based on information received through the communication interface or the user interface, identify drug components included in the identified prescription information, obtain food information based on the identified drug component, and output the obtained food information through the display to provide the same to the user.

## Description

### TECHNICAL FIELD

The present disclosure relates to an artificial intelligence-based system and, more particularly, to a technology for providing customized services in connection with prescription of drugs by portalizing an application of an AI-based green welfare card system that provides data obtained through collection and analysis of user data to affiliated institutions and information on electronic vouchers and customized health management plans to users.

In addition, the present disclosure also relates to a healthcare and welfare system, and more particularly, to an electronic device for analyzing prescription information of a user and providing food information suitable for the corresponding drug components in connection with an AI-based green welfare card system.

### BACKGROUND

The AI-based welfare card system is a next generation support system that aims to manage the health status and life information of users in an integrated manner using artificial intelligence technology, and provide personalized services based on this.

The existing system has a limitation in that support is provided based on information and fragmentary qualification determination scattered among various organizations, making it difficult to provide support corresponding to the actual situation of the demander. In contrast, the AI-based welfare card has an advantage in that it can analyze various public and private data, health/life records of a user, etc., and design more detailed and systematically customized service benefits through this analysis.

Such a system is evolving into an appropriate form for subjects who need continuous health care and welfare support such as the elderly, people with chronic diseases, disabled persons, etc. For example, when a user suffers from a disease such as hypertension or diabetes, it may not only provide welfare benefits but also provide suitable health management information for the corresponding disease, and be linked to subsequent support such as medical expense support, provision of nutritional food, etc. As such, there is increasing interest in technical bases that allow breaking down the boundaries between health management and living welfare, and enable integrated welfare services.

In particular, in the past, medication guidance was provided by a pharmacist or medical staff only once, and dietary management or nutritional information was delivered through separate institutions or platforms.

However, this method has a structure in which information is fragmented and difficult to recognize from the user's point of view, and it is difficult to be put into practical practice. Meanwhile, when an electronic device linked with an AI-based welfare card is used, the user may receive food intake precautions or health functional food recommendation information at the same time as receiving prescription drugs, thereby naturally performing medication guidance and nutrition management in daily life.

### SUMMARY

### TECHNICAL PROBLEMS

The present disclosure provides an electronic device that automatically identifies drug components based on prescription information input from a user or received from a server and obtains food information suitable for the corresponding component or to be avoided, thereby providing the same to the user through a display. This allows us to provide safe and personalized dietary information to users who are currently taking medication.

The objects of the present disclosure are not limited to the above-mentioned purposes, and other objects and advantages of the present disclosure may be understood by the following description, and will become more apparent from embodiments of the present disclosure.

In addition, it will be readily understood that the objects and advantages of the present disclosure may be realized by means and combinations set forth in the claims.

### SOLUTIONS TO THE PROBLEMS

According to an embodiment of the present disclosure, the electronic device may be linked with an AI green welfare card system based on collection and provision of user data to provide a customized service for drug prescription. The electronic device may include a user interface configured to receive user input, a communication interface configured to perform communication connection with a server, a display configured to output visual content, and a processor connected to the user interface, the communication interface, and the display, and capable of performing control operation by executing at least one instruction. The processor may identify prescription information based on information received through the communication interface or the user interface, identify drug components included in the identified prescription information, obtain food information based on the identified drug component, and output the obtained food information through the display to provide the same to the user.

The processor may obtain a prescription image through a camera and identify text included in the obtained prescription image to identify prescription information.

The processor may input the prescription information into an artificial intelligence model to obtain food information corresponding to drug components included in the prescription information based on an outputted value.

The processor may obtain food information corresponding to the identified drug components based on a drug-food interaction database.

The processor may obtain food information including recommended food information that a user is advised to consume with a drug and non-recommended food information that may cause side effects when consumed by the user with the drug based on the identified drug components.

The processor may obtain food information including recommended food information that a user is advised to consume with a drug and non-recommended food information that may cause side effects when consumed by the user with the drug based on the user's health information and personal information.

When user input for selecting at least one of the foods displayed on a display through user interface while outputting food information through the display, is received, the processor may output sales information of the selected food through the display to provide the same to the user.

The processor may output a plurality of seller lists selling the selected food through the display to provide the same to the user, wherein the list may be sorted in display order of the plurality of sellers based on evaluation scores and sales volume.

The processor may sort the display order of the plurality of sellers such that sellers having high evaluation scores and sales volumes are positioned at the top of the list, and sellers having low evaluation scores and sales volumes are positioned at the bottom of the list, and output the list through a display to provide the same to the user.

The processor may collect information on evaluation scores and sales volumes of each of the plurality of sellers, and update a list including the plurality of sellers based on the collected information on the evaluation scores and sales volumes of each of the plurality of sellers at a predetermined period.

The processor may obtain information on a seller selected by the user for purchase, obtain a purchase history database of the user based on the obtained information on the seller, and train a seller recommendation model based on the purchase history database.

The processor may identify the selected food included in the recommended food information when a user input for selecting at least one food from the recommended food information is received through the user interface, input the identified selected food into the seller recommendation model to identify a recommended seller corresponding to the selected food based on an outputted value, generate a seller list including the identified recommended seller, and output the generated seller list through the display to provide the same to the user.

In the method of operating a system according to an embodiment of the present disclosure, the server may identify prescription information based on information received from an external device or a user terminal, identify drug components included in the identified prescription information, and obtain food information based on the identified drug components. The server may transmit the obtained food information to the user terminal, and the user terminal may output the food information received from the server to provide the same to the user.

### ADVANTAGEOUS EFFECTS OF INVENTION

According to the present disclosure, drug components are automatically extracted based on prescription information of a user and food information related to the corresponding component is searched for and provided, thereby enabling users to perform diet management more safely and efficiently by themselves. In particular, providing information about foods that should be avoided or nutrients recommended when taking specific drugs maximizes the effect of drug intake and prevents side effects.

In addition, since the system is linked to an AI green welfare card system and provides customized information based on user data, it can be extended to a welfare-linked service suitable for individual situations. The present technology may be used in various fields such as medication guidance, electronic prescription systems, digital healthcare platforms, etc.

In conclusion, the present disclosure can be utilized in various fields such as public welfare, private partnership platforms, and AI-based services.

### BRIEF DESCRIPTION OF DRAWINGS

Aspects, features and advantages of certain embodiments of the present disclosure will become more apparent with the following description in conjunction with the accompanying drawings.
FIG. 1 is a diagram illustrating an example configuration of a system including a server, a user terminal, and an affiliated institution terminal according to one embodiment of the present disclosure.
FIG. 2 is a block diagram illustrating the configuration of a user terminal according to an embodiment of the present disclosure.
FIG. 3 is a block diagram illustrating the configuration of a server according to an embodiment of the present disclosure.
FIG. 4 is a block diagram illustrating the configuration of an affiliated institution terminal according to an embodiment of the present disclosure.
FIG. 5 is a sequence diagram for describing representative operations of the system including the server, user terminal, and affiliated institution terminal according to an embodiment of the present disclosure.
FIG. 6 is a diagram illustrating an operation of photographing a prescription image with an electronic device using an OCR method to identify text included in the prescription according to an embodiment of the present disclosure.
FIG. 7 is a block diagram illustrating an operation in which the electronic device inputs prescription information into the artificial intelligence model to obtain information on recommended foods suitable for consumption together with the drug components included in the prescription and non-recommended foods unsuitable for consumption together with, or capable of causing side effects when consumed together with, the drug components included in the prescription, according to an embodiment of the present disclosure.
FIG. 8 is a block diagram illustrating an operation in which the electronic device obtains information on recommended foods suitable for consumption together with the drug components included in the prescription and non-recommended foods unsuitable for consumption together with, or capable of causing side effects when consumed together with, the drug components included in the prescription, based on the prescription information and drug-food interaction database according to an embodiment of the present disclosure.
FIG. 9 is a block diagram illustrating an operation in which the electronic device obtains information on recommended foods suitable for consumption together with the drug components included in the prescription and non-recommended foods unsuitable for consumption together with, or capable of causing side effects when consumed together with, the drug components included in the prescription, based on the prescription information, health information and personal information according to an embodiment of the present disclosure.
FIG. 10 is a flowchart illustrating an operation of the electronic device according to an embodiment of the present disclosure.
FIG. 11 is a diagram illustrating a screen on which the electronic device outputs, through a display, a seller list selling recommended foods for drug components included in the prescription information to provide the same to the user according to an embodiment of the present disclosure.
FIG. 12 is a diagram illustrating a screen on which the electronic device outputs, through a display, a seller list selling recommended health functional foods for the drug components included in prescription information to provide the same to the user according to an embodiment of the present disclosure.
FIG. 13 is a diagram illustrating an operation in which the electronic device inputs a food item of a user into a seller recommendation model learned based on a user purchase history to obtain a seller list corresponding to the food item according to an embodiment of the present disclosure.
FIG. 14 is a diagram illustrating a screen on which the electronic device outputs a recommended seller list through the display to provide the same to the user according to an embodiment of the present disclosure.

### MODE FOR CARRYING OUT THE INVENTION

Prior to describing the present disclosure in detail, a description method of the present specification and drawings will be described.

First of all, the terms used in this specification and claims have been selected considering functions in various embodiments of the present disclosure. However, these terms may vary depending on the intention of a person skilled in the art or legal or technical interpretation and appearance of new technology. In addition, some terms are arbitrary terms selected by an applicant. These terms may be interpreted as defined herein, and if there is no specific definition of a term, the terms may also be interpreted based on the overall content of the specification and common technical knowledge in the relevant technical field.

In addition, the same reference numerals or signs in each of the drawings attached to this specification indicate parts or components that perform substantially the same function. For convenience of description and understanding, the same reference numerals or signs are used even in different embodiments. That is, although all elements having the same reference numerals in a plurality of drawings are illustrated, it does not mean that the plurality of drawings represent one embodiment.

In addition, in the specification and claims, to distinguish between elements, terms including ordinal numbers such as "first", "second" may be used. Such ordinal numbers are used to distinguish identical or similar elements from each other, and the meaning of a term should not be limited by using such ordinal numbers. For example, an element combined with such ordinal number is not restricted to its use order or arrangement order by the number. If necessary, each ordinal number may be replaced with another one.

In the present specification, a singular form includes plural forms unless it is clear from context that the singular form is not to be interpreted as the plural form. In this application, terms such as "include" or "comprise" are intended to indicate that features, numbers, steps, operations, elements, parts, or combinations thereof described in the specification exist, but do not preclude the existence or addition of one or more other features, numbers, steps, operations, elements, parts, or combinations thereof.

In the present disclosure, terms such as "module", "unit", and "part" refer to a component that performs at least one function or operation, which may be implemented by hardware or software, or a combination of hardware and software. In addition, unless each of the plurality of modules, units, and parts needs to be implemented with separate specific hardware, they may be integrated into at least one module or chip and implemented in at least one processor.

In addition, in the embodiments of the present disclosure, when a certain part is connected to another part, it includes not only direct connection but also indirect connection through other media. In addition, the meaning that a certain part comprises a specific component means that it may further include other components without excluding other components unless otherwise specified.

In this disclosure, the expression "at least one of a, b or c" may refer to "a", "b", "c", "a and b", "a and c", "b and c", "all of a, b and c", or variations thereof.

The terms used in the present disclosure are selected from general terms currently widely used considering functions thereof, but may vary depending on intentions of those skilled in the art or precedents, emergence of new technologies, etc. In addition, there are terms arbitrarily selected by an applicant, and meanings thereof will be described in detail in corresponding description sections. Therefore, the terms used in the present disclosure should be defined based on their meanings and the overall content of the present disclosure, not merely as simple names of terms.

The singular forms may include the plural unless the context clearly indicates otherwise. The terms used herein, including technical and scientific terms, may have the same meaning as commonly understood by those of ordinary skill in the art to which this disclosure pertains. In addition, although terms containing ordinal numbers such as first, second, etc., are used to describe various components, these components should not be limited by such terms. These terms are only used to distinguish one component from another.

Throughout the specification, when an element is referred to as including a certain component, it should be understood that the element may further include other components without being limited thereto unless otherwise specified. In addition, terms such as "part", "module" used in the specification refer to units processing at least one function or operation, which can be implemented by hardware, software, or a combination thereof.

Hereinafter, embodiments of the present disclosure will be described in detail with reference to the accompanying drawings so that those skilled in the art to which the present invention pertains can easily implement the same. However, it should be understood that the present disclosure is not limited to the specific embodiments set forth herein. In addition, parts unrelated to description are omitted from the drawings for clarity, and like numbers refer to like elements throughout the specification.

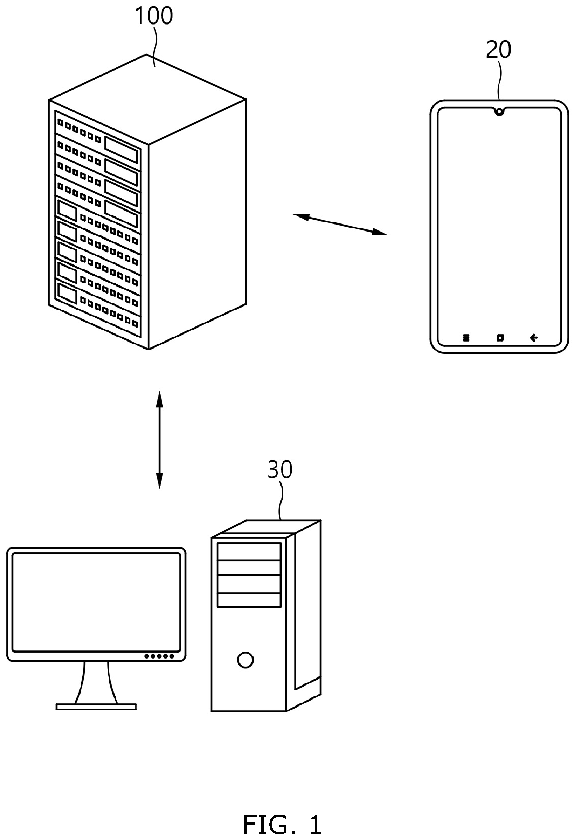

FIG. 1 is a diagram illustrating an example configuration of a system including a server 100, a user terminal 20, and an affiliated institution terminal 30 according to one embodiment of the present disclosure.

Referring to FIG. 1, the server 100 may be a file transfer protocol (FTP) server, a web server, a database server, or a cloud server, but is not limited thereto.

The server 100 may perform one or more functions and operations, and may be implemented as a single device or distributed across multiple devices to implement each function and operation.

The user terminal 20 may be a smartphone, a tablet personal computer (tablet PC), a laptop personal computer (laptop PC), a desktop PC, a wearable device (e.g., smart watch, smart TV, set-top box, console, virtual reality (VR) and augmented reality (AR) dedicated terminal (e.g., head mounted device (HMD)), smart home device, industrial special terminal, vehicle terminal, etc.), but is not limited thereto.

The user terminal 20 may perform one or more functions and operations, and the functions and operations may be implemented by a single device or distributed across multiple devices.

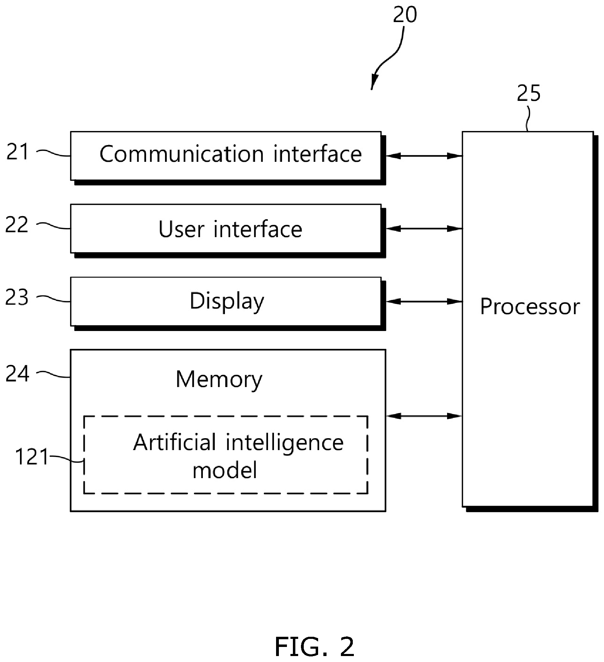

FIG. 2 is a block diagram illustrating the configuration of a user terminal 20 according to an embodiment.

Referring to FIG. 2, the user terminal 20 may include a communication interface 21, a user interface 22, a display 23, a memory 24, and a processor 25, but is not limited thereto, and may further include various configurations or omit some configurations.

The communication interface 21 may include at least one of a wireless communication interface, a wired communication interface, and an input/output interface. The wireless communication interface may perform communication with various external devices using a wireless communication technology or a mobile communication technology. Examples of the wireless communication technology may include Bluetooth, Bluetooth Low Energy, CAN communication, Wi-Fi, Wi-Fi Direct, ultrawide band (UWB), ZigBee, Infrared Data Association (IrDA), or Near Field Communication (NFC), and examples of the mobile communication technology may include 3GPP, Wi-Max, Long Term Evolution (LTE), and 5G.

The wireless communication interface may be implemented using an antenna for transmitting electromagnetic waves to the outside or receiving electromagnetic waves from the outside, a communication chip, and a substrate.

The wired communication interface may perform communication with various devices based on a wired communication network. Herein, the wired communication network may be implemented using a physical cable such as a pair cable, a coaxial cable, an optical fiber cable or an Ethernet cable.

One of the wireless communication interface and the wired communication interface may be omitted according to an embodiment. Thus, the server 100 may include only a wireless communication interface or only a wired communication interface. In addition, the server 100 may include an integrated communication interface 21 that supports both a wireless connection through the wireless communication interface and a wired connection through the wired communication interface.

The processor 25 may perform communication connection with the external device, the server 100 and the affiliated institution terminal 30 through the communication interface 21 to transmit or receive various data, signals, etc.

Herein, various information, signals, data, etc. may be prescription P information, drug information, medicine information, health information, personal information, recommended food R information F about the drug components included in the prescription P, non-recommended food NR information F, and a seller list M for the recommended foods R, but are not limited thereto.

The user interface 22 may include a button, a lever, a switch (D3), a touch-type interface, a camera, a microphone, a sensor (e.g., a gesture sensor, a motion sensor, a touch sensor, or a biometric sensor), and an input interface, but is not limited thereto.

The processor 25 may receive user input for data processing and data entry through the user interface 22.

The processor 25 may receive various user inputs through the user interface 22. Herein, the user input may be prescription P information, drug information, medicine information, health information, personal information, recommended food R information F corresponding to drug components included in the prescription P, non-recommended food NR information F, and the recommended food R sellers, but is not limited thereto.

If the user interface 32 is a camera, then the processor 35 may obtain a prescription P image through the camera.

The camera may be a device for capturing still images and moving pictures. According to an embodiment of the present disclosure, the camera may include one or more lenses (e.g., convex lens, concave lens, spherical lens, planar lens, wide angle lens) that refracts and collects light or spreads light, an image sensor (e.g., charge-coupled device (CCD), complementary metal-oxide semiconductor (CMOS)) that converts light into electric charges to obtain an image, an image signal processor, or a flash. In addition to the above, the camera may include an aperture, a viewfinder, a zebra device configured to detect whether an image is overexposed through a CCD inside the camera, etc.

The processor 25 may sense light in the visible region through a camera to obtain an RGB image. The camera may sense light in the infrared region to obtain an infrared image.

The display 23 may include various types of display panels such as a liquid crystal display (LCD) panel, an organic light emitting diode (OLED) panel, an active matrix-organic light emitting diode (AM-OLED), a liquid crystal on silicon (LCoS), a quantum dot light-emitting diode (QLED), and a digital light processing (DLP) panel, a plasma display panel (PDP), an inorganic LED panel, a Micro LED panel, etc., but is not limited thereto. Meanwhile, the display 23 may be configured to form a touch screen with the touch panel or may be formed of a flexible panel.

The display 23 may be implemented in a square or rectangular shape of 2D, but is not limited thereto and may be implemented in various shapes such as a circle, polygon, 3D object, etc.

The display 23 may be disposed on one area of the surface of the user terminal 20, but is not limited thereto and may be implemented as a three-dimensional hologram projected onto a three-dimensional space or a projection form projected onto a two-dimensional plane.

The processor 25 may output, through the display 23, the content visualizing the prescription P image, the prescription P information, the text included in the prescription P, the drug components included in the prescription P, the recommended foods R and the non-recommended foods NR corresponding to the drug components included in the prescription P, and the seller list M of the recommended foods R, to provide the same to the user.

However, the processor 25 is not limited thereto and may output various GUIs, images, videos, or texts through the display 23 to provide the same to the user.

The memory 24 temporarily or non-temporarily stores various programs and data, and transfers the stored information to the processor 25 according to a call of the processor 25. In addition, the memory 24 may store various pieces of information required for an operation, processing, or control operation of the processor 25 in an electronic format.

The memory 24 may include at least one of a main storage and an auxiliary storage. The main storage may be implemented using a semiconductor storage medium such as ROM and/or RAM. The ROM may include, for example, a conventional ROM, an EPROM, an EEPROM, and/or a MASK-ROM. The RAM may include, for example, DRAM and/or SRAM. The auxiliary storage may be implemented by using at least one storage medium capable of permanently or semi-permanently storing data, such as a flash memory device, a secure digital (SD) card, a solid state drive (SSD), a hard disc drive (HDD), a magnetic drum, an optical media such as compact disc (CD), a digital versatile disc (DVD), a laser disc, a magnetic tape, a magneto-optical disk, and/or a floppy disk.

The memory 24 may store the artificial intelligence model 121. Herein, the artificial intelligence model 121 may be an artificial intelligence model 121, a deep learning model, or a neural network model, but is not limited thereto.

The artificial intelligence model 121 may be a model that outputs information on recommended foods or non-recommended foods based on the drug components included in the prescription P when the prescription P information is input.

The memory 24 may store an artificial intelligence model, output data obtained based on input data inputted to the artificial intelligence model, usage information obtained based on the output data, and reconstruction information obtained based on the usage information for reconstructing the artificial intelligence model.

The artificial intelligence model 121 may be constructed in consideration of an application field of the recognition model, a purpose of learning, or computer performance of the device. In addition, the artificial intelligence model may be, for example, a model based on a neural network.

The artificial intelligence model may be designed to simulate the structure of a human brain on a computer. The artificial intelligence model may include a plurality of network nodes having weights and simulating neurons of a human neural network. The plurality of network nodes may form connection relationships with each other in order to simulate synaptic activity by which neurons exchange signals through synapses.

The artificial intelligence model may include a neural network model or a deep learning model developed from the neural network model. In the deep learning model, a plurality of network nodes are located at different depths (or layers) and exchange data according to a convolution connection relationship. For example, models such as a deep neural network (DNN), recurrent neural network (RNN), and bidirectional recurrent deep neural network (BRDNN) may be used as an artificial intelligence model without being limited thereto.

The deep learning model may include a plurality of artificial neural network layers. The artificial neural network may be one of a deep neural network (DNN), a convolutional neural network (CNN), a recurrent neural network (RNN), a restricted Boltzmann machine (RBM), a deep belief network (DBN), a bidirectional recurrent deep neural network (BRDNN), a deep Q-network, or one of the combinations of two or more of the above, but is not limited to the examples mentioned above. In addition to the hardware structure, the artificial intelligence model 121 may additionally or alternatively include a software structure.

The deep learning model may include a learning algorithm. For example, the deep learning model may include supervised learning, unsupervised learning, semi-supervised learning or reinforcement learning, but is not limited thereto. The artificial intelligence model 121 may include a plurality of artificial neural network layers.

In addition to the above, the memory 24 may store a prescription P image, prescription P information, drug information, medicine information, health information, personal information, recommended food R information F, non-recommended food NR information F, and a seller list M of the recommended foods R, but is not limited thereto.

The processor 25 controls the overall operation of the server 100. Specifically, the processor 25 is connected to the configuration of the server 100 including the memory 24 as described above, and may control the overall operation of the server 100 by executing at least one instruction stored in the memory 24 as described above. In particular, the processor 25 may be implemented with a single processor or a plurality of processors.

The processor 25 may be implemented in various ways. For example, the one or more processors 25 may include at least one of a central processing unit (CPU), a graphics processing unit (GPU), an accelerated processing unit (APU), many integrated cores (MIC), a digital signal processor (DSP), a neural processing unit (NPU), a hardware accelerator, or a machine learning accelerator. The one or more processors 25 may control any one or any combination of other components of the server 100 and perform operations related to communication or data processing. The one or more processors 25 may execute one or more programs or instructions stored in the memory 24. For example, the one or more processors 25 may execute the one or more instructions stored in the memory 24 to perform a method according to an embodiment of the present disclosure.

In embodiments of the present disclosure, the processor 25 may refer to a system on chip (SoC) in which one or more processors 25 and other electronic components are integrated, a single core processor, a multi-core processor, or a core included in a single core processor or a multi-core processor, where the core may be implemented as a CPU, GPU, APU, MIC, DSP, NPU, hardware accelerator, or machine learning accelerator, but embodiments of the present disclosure are not limited thereto.

The processor 25 may perform optical character recognition (OCR) on the prescription P image to identify text included in the prescription P image and identify drug components included in the prescription P information based on the identified text.

The processor 25 may obtain information on the recommended foods R or the non-recommended foods matched to the drug components included in the prescription P based on the outputted value obtained by inputting the prescription P information into the artificial intelligence model 121.

In addition to the above, the processor 25 may generate a seller list M that sell the recommended foods R to be provided to the user terminal 20.

However, this is not limited thereto, and the processor 25 may perform various operations of controlling the configuration of the user terminal 20 described above by executing at least one instruction.

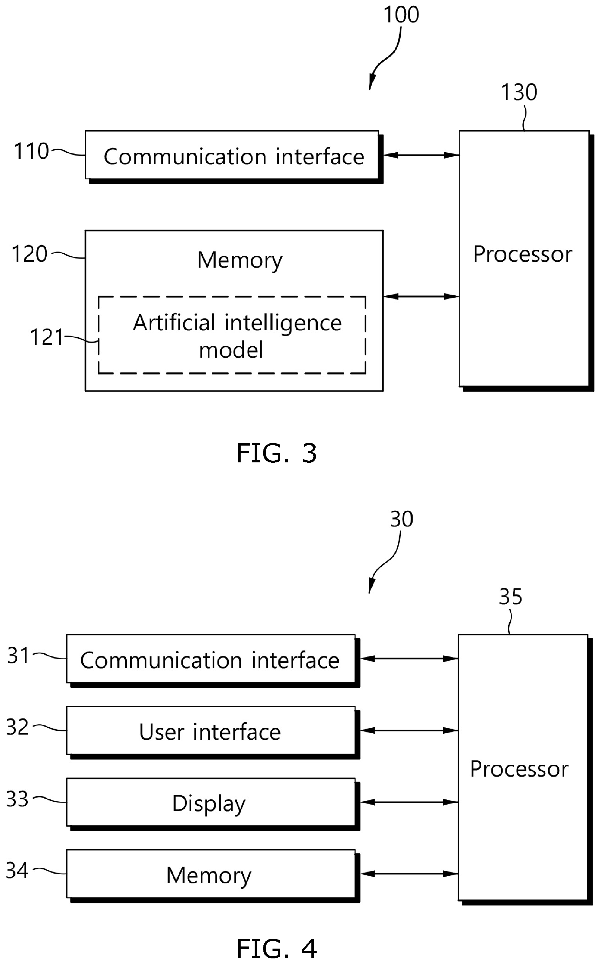

FIG. 3 is a block diagram illustrating the configuration of a server 100 according to an embodiment of the present disclosure.

Referring to FIG. 3, the server 100 may include a communication interface 110, a memory 120, and a processor 130. However, this is not limited thereto, and may further include other components or omit some components.

The communication interface 110 may include at least one of a wireless communication interface, a wired communication interface, and an input/output interface. The wireless communication interface may perform communication with various external devices using a wireless communication technology or a mobile communication technology. Examples of the wireless communication technology may include Bluetooth, Bluetooth Low Energy, CAN communication, Wi-Fi, Wi-Fi Direct, ultrawide band (UWB), ZigBee, Infrared Data Association (IrDA), or Near Field Communication (NFC), and examples of the mobile communication technology may include 3GPP, Wi-Max, Long Term Evolution (LTE), and 5G.

The wireless communication interface may be implemented using an antenna for transmitting electromagnetic waves to the outside or receiving electromagnetic waves from the outside, a communication chip, and a substrate.

The wired communication interface may perform communication with various devices based on a wired communication network. Herein, the wired communication network may be implemented using a physical cable such as a pair cable, a coaxial cable, an optical fiber cable or an Ethernet cable.

The wireless communication interface and the wired communication interface may be omitted according to an embodiment. Thus, the server 100 may include only a wireless communication interface or only a wired communication interface. In addition, the server 100 may include an integrated communication interface 110 that supports both a wireless connection through the wireless communication interface and a wired connection through the wired communication interface.

The server 100 is not limited to a case in which the communication interface 110 performs communication connection in one way and may include a plurality of communication interfaces 110 that perform communication connection in a plurality of ways.

The processor 130 may perform communication connection with the user terminal 20 or the affiliated institution terminal 30 through the communication interface 110 to transmit or receive a prescription P image, prescription P information, drug information, medicine information, health information, personal information, and so on.

In addition to the above, the processor 130 may perform communication connection with the user terminal 20 or the affiliated institution terminal 30 through the communication interface 110 to transmit or receive recommended food R information F, non-recommended food NR information F, a seller list M of recommended foods R, and so on.

The memory 120 temporarily or non-temporarily stores various programs and data, and transfers the stored information to the processor 130 according to a call of the processor 130. In addition, the memory 120 may store various pieces of information required for an operation, processing, or control operation of the processor 130 in an electronic format.

The memory 120 may include at least one of a main storage and an auxiliary storage. The main storage may be implemented using a semiconductor storage medium such as ROM and/or RAM. The ROM may include, for example, a conventional ROM, an EPROM, an EEPROM, and/or a MASK-ROM. The RAM may include, for example, DRAM and/or SRAM. The auxiliary storage may be implemented by using at least one storage medium capable of permanently or semi-permanently storing data, such as a flash memory device, a secure digital (SD) card, a solid state drive (SSD), a hard disc drive (HDD), a magnetic drum, an optical media such as a compact disc (CD), a digital versatile disc (DVD), a laser disc, a magnetic tape, a magneto-optical disk, and/or a floppy disk.

The memory 120 may store the artificial intelligence model 121. Herein, the artificial intelligence model 121 may be an artificial intelligence model 121, a deep learning model, or a neural network model, but is not limited thereto.

The artificial intelligence model 121 may be a model that outputs information on recommended foods or non-recommended foods based on the drug components included in the prescription P when the prescription P information is input.

The memory 120 may store an artificial intelligence model 121, output data obtained based on input data inputted to the artificial intelligence model 121, usage information obtained based on the output data, and reconstruction information obtained based on the usage information for reconstructing the artificial intelligence model.

In addition to the above, the memory 120 may store a prescription P image, prescription P information, drug information, medicine information, health information, personal information, recommended food R information F, non-recommended food NR information F, and a seller list M of the recommended foods R, but is not limited thereto.

The processor 130 controls the overall operation of the server 100. Specifically, the processor 130 is connected to the configuration of the server 100 including the memory 120 as described above, and may control the overall operation of the server 100 by executing at least one instruction stored in the memory 120 as described above. In particular, the processor 130 may be implemented with a single processor or a plurality of processors.

The processor 130 may be implemented in various ways. For example, the one or more processors 130 may include at least one of a central processing unit (CPU), a graphics processing unit (GPU), an accelerated processing unit (APU), many integrated cores (MIC), a digital signal processor (DSP), a neural processing unit (NPU), a hardware accelerator, or a machine learning accelerator. The one or more processors 130 may control any one or any combination of other components of the server 100 and perform operations related to communication or data processing. The one or more processors 130 may execute one or more programs or instructions stored in the memory 120. For example, the one or more processors 130 may execute the one or more instructions stored in the memory 120 to perform a method according to an embodiment of the present disclosure.

In embodiments of the present disclosure, the processor 130 may refer to a system on chip (SoC) in which one or more processors 130 and other electronic components are integrated, a single core processor, a multi-core processor, or a core included in a single core processor or a multi-core processor, where the core may be implemented as a CPU, GPU, APU, MIC, DSP, NPU, hardware accelerator, or machine learning accelerator, but embodiments of the present disclosure are not limited thereto.

The processor 130 may perform optical character recognition (OCR) on the prescription P image to identify text included in the prescription P image and identify drug components included in the prescription P information based on the identified text.

The processor 130 may obtain information on the recommended foods R or the non-recommended foods matched to the drug components included in the prescription P based on the outputted value obtained by inputting the prescription P information into the artificial intelligence model 121.

In addition to the above, the processor 130 may generate a seller list M that sell the recommended foods R to be provided to the user terminal 20.

However, this is not limited thereto, and the processor 130 may perform various operations of controlling the configuration of the server 100 described above by executing at least one instruction.

The affiliated institution terminal 30 may be an organization affiliated with the system for implementing the present embodiment, such as a hospital, medical center, pharmacy, pharmaceutical company, healthcare company, or the like, but is not limited thereto.

The affiliated institution terminal 30 may be, for example, a smartphone, a tablet personal computer (tablet PC), a laptop personal computer (laptop PC), a desktop PC, a wearable device (e.g., a smart watch), a smart TV, a set-top box, a console, a virtual reality (VR) and augmented reality (AR) dedicated terminal (e.g., a head mounted device (HMD)), a smart home device, an industrial special terminal, or a vehicle terminal, but is not limited thereto.

The affiliated institution terminal 30 may perform one or more functions and operations, and the functions and operations may be implemented by a single device or distributed in multiple devices.

FIG. 4 is a block diagram illustrating the configuration of an affiliated institution terminal 30 according to an embodiment of the present disclosure.

Referring to FIG. 4, the affiliated institution terminal 30 may include a communication interface 31, a user interface 32, a display 33, a memory 34, and a processor 35, but is not limited thereto.

The detailed functions, operations, and configurations of the affiliated institution terminal 30 may be identical to those of the user terminal 20 described above, but are not limited thereto.

The processor 35 may perform communication connection with the server 100 or the user terminal 20 through the communication interface 31 to transmit and receive various information, signals, data, etc.

Herein, various information, signals, data, etc. may be prescription P information, drug information, medicine information, health information, personal information, etc., but is not limited thereto.

The processor 35 may receive various user inputs through the user interface 32. Herein, the user input may be prescription P information, drug information, medicine information, health information, personal information, recommended food R information F corresponding to drug components included in the prescription P, non-recommended food NR information F, and the recommended food R sellers, but is not limited thereto.

When the user interface 32 is a camera, the processor 35 may obtain a prescription P image through the camera.

The processor 35 may output various visual contents through the display 33 to provide the same to the user.

Herein, the visual content may be prescription P information, drug information, medicine information, health information, personal information, recommended food R, and non-recommended food NR information F matched with drug components, but is not limited thereto.

The processor 35 may store prescription P information, drug information, medicine information, health information, personal information, and the like in the memory 34. However, this is not limited thereto, and the processor 35 may store various information, data, signals, and the like in the memory 34.

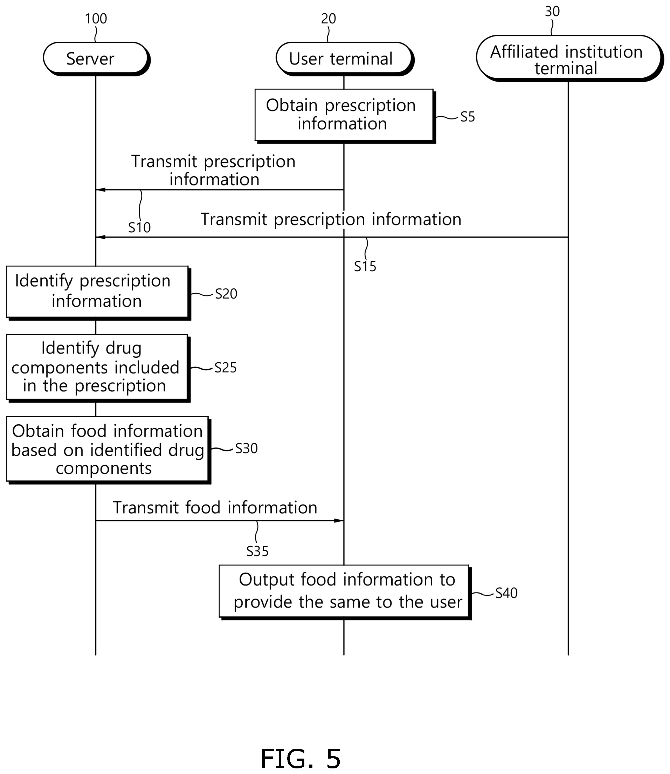

FIG. 5 is a sequence diagram for describing representative operations of the system including the server 100, user terminal, and affiliated institution terminal 30 according to an embodiment of the present disclosure.

Referring to FIG. 5, the user terminal may obtain prescription P information (S5). The user terminal may transmit the obtained prescription P information to the server 100 (S10).

At this time, the user terminal may obtain a prescription P image through a camera and identify text included in the obtained prescription P image to identify prescription P information.

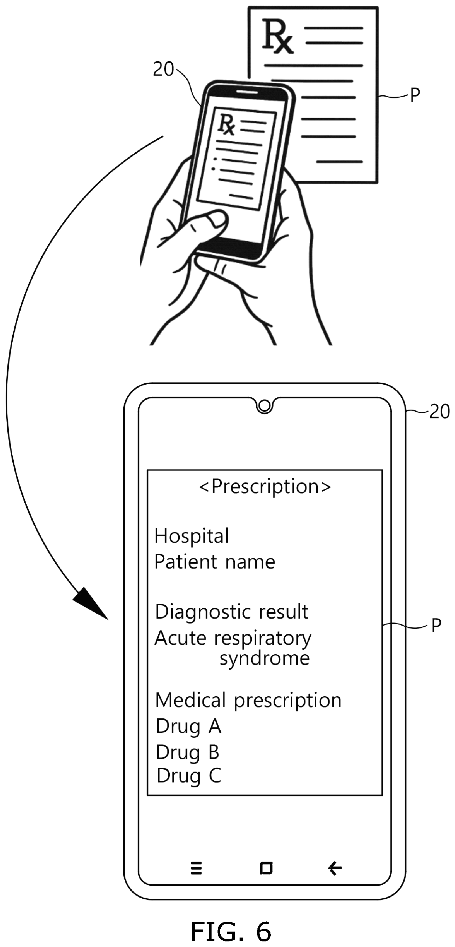

FIG. 6 is a diagram illustrating an operation of photographing a prescription P image with an electronic device 20 using an OCR method to identify text included in the prescription P according to an embodiment of the present disclosure.

Referring to FIG. 6, the prescription P information includes data such as a patient name, drug name, dosage, and number of doses, and text identification may be generally performed through an optical character recognition (OCR) technology.

Optical character recognition (OCR) may be a technology for automatically recognizing character data such as letters and numbers included in an image or scanned document and converting the recognized characters into digital text.

The basic operation principle of the OCR consists of a process for detecting an area including text in an image and then segmenting individual characters within the corresponding area to determine what each character is. Initially, it was mainly used to recognize standardized printed characters from black-and-white documents input by a scanner, but now it can recognize photographs taken with a smartphone camera, handwritten texts, tables and even signs or license plates in natural images.

The user terminal may perform an image preprocessing operation. This is a step of removing noise from the image and correcting a tilted or dark background to facilitate character recognition.

The user terminal may perform a character recognition operation. In this process, the character patterns are compared and the corresponding character is predicted using an artificial intelligence-based algorithm.

The user terminal may perform a postprocessing operation. Incorrectly recognized characters are corrected or the text is organized into natural text by understanding the context. Recently, a language model is used in the postprocessing process to automatically correct words according to the context.

The OCR technology may be implemented in a deep learning-based OCR method as well as the conventional pattern recognition method. Representative models include convolutional recurrent neural network (CRNN), an attention-based model, and a transformer-based recognition network, which ensure high recognition rate even for complex sentence structures or various fonts. In particular, it is possible to accurately extract text by considering the meaning of sentences in combination with natural language processing (NLP) technology.

The user terminal may transmit the text included in the identified prescription P image to the server 100.

At this time, the text may include information on a hospital name, a medical institution name, a patient name, drug prescription information, and disease information of the patient, but is not limited thereto.

The camera is configured to convert visual information into digital images and may be used to collect medical information written on a paper document. For example, the internal camera of a smartphone or a lens included in an OCR scanner may correspond thereto. However, this is not limited thereto.

The user terminal may identify text included in the obtained prescription P image to identify information of the prescription P. The prescription P information includes data such as a patient name, drug name, dosage, and number of doses, and the affiliated institution terminal 30 may transmit prescription P information, a prescription P image, text included in the prescription P, and drug prescription information to the server 100 (S15).

The user terminal and the affiliated institution terminal 30 do not necessarily both transmit prescription P information, a prescription P image, and drug prescription information to the server 100, and only one of the two devices may transmit prescription P information, a prescription P image, and drug prescription information to the server 100.

The server 100 may identify the prescription P information based on the received information from the user terminal or the affiliated institution terminal 30 (S20).

At this time, when the prescription P image is received, the server 100 may identify text included in the prescription P image to identify prescription P information.

The prescription P information includes data such as a patient name, drug name, dosage, and number of doses, and text identification may be generally performed through an optical character recognition (OCR) technology.

The server 100 may perform an image preprocessing operation. This is a step of removing noise from the image and correcting a tilted or dark background to facilitate character recognition.

The server 100 may perform a character recognition operation. In this process, the character patterns are compared and the corresponding character is predicted using an artificial intelligence-based algorithm.

The server 100 may perform a postprocessing operation. Incorrectly recognized characters are corrected or the text is organized into natural text by understanding the context. Recently, a language model is used in the postprocessing process to automatically correct words according to the context.

The server 100 may obtain the food information F corresponding to the drug components included in the prescription P based on a outputted value obtained by inputting the prescription P information into an artificial intelligence model 121 (e.g., a food recommendation model).

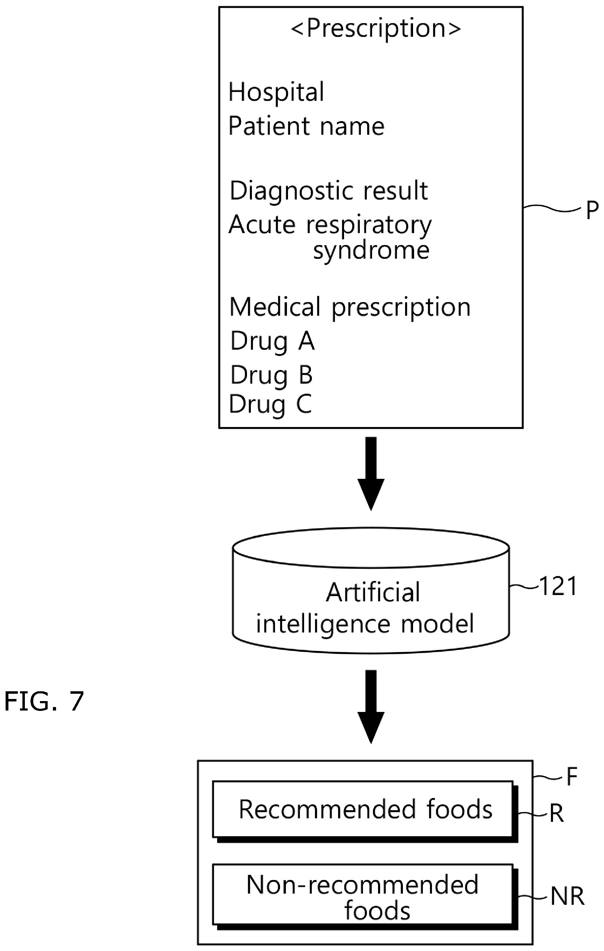

FIG. 7 is a block diagram illustrating an operation in which the electronic device 20 inputs prescription P information into the artificial intelligence model 121 to obtain information on recommended foods suitable for consumption together with the drug components included in the prescription P and non-recommended foods unsuitable for consumption together with, or capable of causing side effects when consumed together with, the drug components included in the prescription P, according to an embodiment of the present disclosure.

Referring to FIG. 7, the server 100 may obtain food information F corresponding to drug components included in prescription P information based on an outputted value by inputting prescription P information into the artificial intelligence model 121.

The artificial intelligence model 121 is an algorithm system that performs prediction or classification of input values based on data and may be a model used for prediction such as drug interaction or nutrient analysis in the medical and food fields.

The food information F may be data on interaction with the drug component, absorption promotion or inhibition effect, foods to avoid when taking the drug, etc., and the food information F may be information on recommended foods R that can have a positive effect on the body when taken with the drug components and non-recommended foods NR that cause negative effects or side effects to the body when taken with the drug components.

In addition, the food information F may include foods to be avoided in connection with drug administration of a user, ingredients that can increase or decrease the effect of drugs, advice on diet, and so on. For example, there is information indicating that grapefruit may affect the metabolism of some hyperlipidemia drugs, and isoniazid should be taken with restriction on high-fat foods, which may be used for personalized medication guidance, but is not limited thereto.

For example, there are interactions between warfarin and green vegetables containing a large amount of vitamin K, or calcium supplements and dairy products, which are used to provide medication guidance according to the influence of specific foods on drug efficacy.

According to various embodiments of the present disclosure, the present disclosure relates to an artificial intelligence based system that predicts interactions between drugs and foods, and recommends appropriate food information to a user based on the prediction. More specifically, the present disclosure relates to an electronic device 20 capable of analyzing drug-food interaction data and user health data in an integrated manner by using a natural language processing model based on a transformer, and providing food recommendation information to a user based on the analysis result.

The electronic device 20 receives a Micromedex based drug-food interaction database and individual user health data as input values to predict the interaction between drugs and food. The health data of a user includes, for example, allergy history, taken medications, diagnosed diseases, dietary habits, etc., which may be collected through an input interface of the electronic device 20 or an external medical information server.

The electronic device 20 drives a natural language processing model based on the received data according to a transformer structure. Herein, the transformer refers to a deep learning model that dynamically calculates relationships between elements in a sequence and effectively reflects contextual dependency based on an attention mechanism. The model may identify semantic relationships between food items corresponding to each drug components and evaluate interaction risks accordingly.

The electronic device 20 precisely predicts whether the active ingredients of a drug have high possibility to interact with specific food ingredients by using an attention mechanism. The predicted interaction information is scored and classified as a contraindicated food if it exceeds a predetermined standard. At the same time, the electronic device 20 selects food information having low risk of interaction or neutralization effect and presents the selected food information to the user as recommended foods.

The electronic device 20 uses supervised learning and reinforcement learning in parallel as the learning algorithm. The supervised learning learns data having a correct label based on a drug-food interaction dataset, and the reinforcement learning continuously improves recommendation accuracy according to user feedback (e.g., whether an adverse reaction occurs after actual ingestion).

In terms of technical effects, the electronic device 20 may improve accuracy in predicting drug-food interactions by about 20% or more compared to a conventional rule-based system and reduce processing time for food recommendation by an average of 50% or more. In addition, since customized dietary guidelines based on drug components can be provided, user's compliance with medication and health management efficiency may be greatly improved.

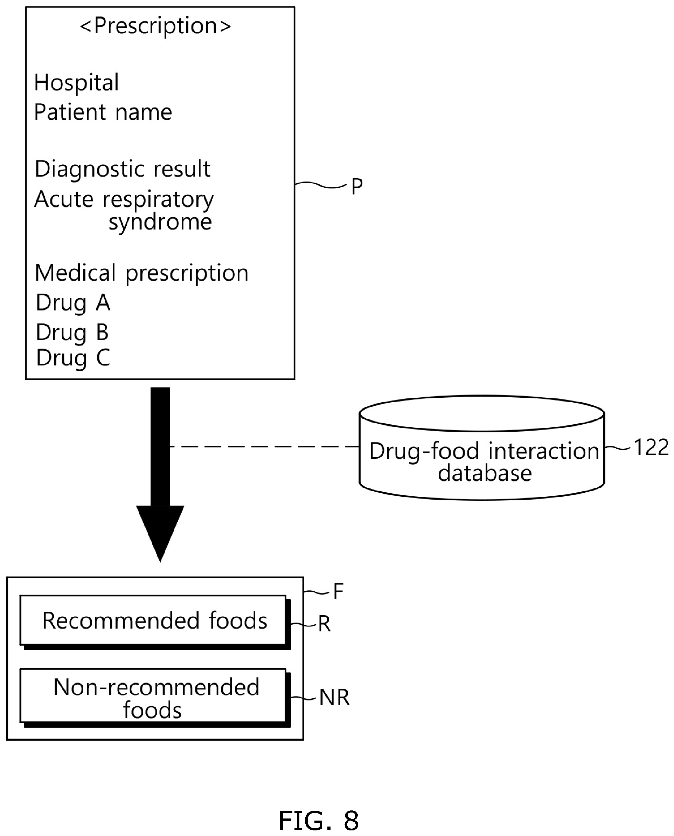

FIG. 8 is a block diagram illustrating an operation in which the electronic device 20 obtains information on recommended foods R suitable for consumption together with the drug components included in the prescription P and non-recommended foods NR unsuitable for consumption together with, or capable of causing side effects when consumed together with, the drug components included in the prescription P based on the prescription P information and the drug-food interaction database 122 according to an embodiment of the present disclosure.

Referring to FIG. 8, the server 100 may obtain food information F corresponding to the identified drug components based on the drug-food interaction database 122.

The drug-food interaction database 122 is a set of information that stores the relationship between specific drugs and specific foods in an organized manner, including food-related data that affect drug absorption rate, metabolism, efficacy, or side effects.

For example, there are Micromedex's Drug-Food Interactions and the FDA's Drug Interaction Database, which can be used to automatically extract contraindicated foods or recommended foods for each drug component, but are not limited thereto.

The server 100 may obtain food information F including recommended food information that a user is advised to consume with a drug and non-recommended food information that may cause side effects when consumed by the user with the drug based on the identified drug components based on the identified drug components.

The identified drug component is an active ingredient of the medicine specified in the prescription P and is a main reference for affecting how the corresponding component acts in the body, metabolic pathways, excretion characteristics, etc. For example, drug components such as 'acetaminophen' and 'warfarin' may be included, and interactions with foods may be analyzed based thereon, but is not limited thereto.

The recommended food information refers to a food that is good to take together with a drug in order to increase the effect of the drug or prevent side effects such as gastrointestinal disorders. For example, there are orange juice which helps absorption when taking an iron supplement, and soft high-protein diet which aids digestion when taking a digestive enzyme agent, but are not limited thereto.

The non-recommended food information is a food that may interfere with drug metabolism or cause side effects, for example, spinach rich in vitamin K when warfarin is taken, and the interaction between grapefruit and some hyperlipidemia treatment drugs. However, this is not limited thereto.

The server 100 may obtain food information F including recommended food information that a user is advised to consume with a drug and non-recommended food information that may cause side effects when consumed by the user with the drug based on the user's health information and personal information 123.

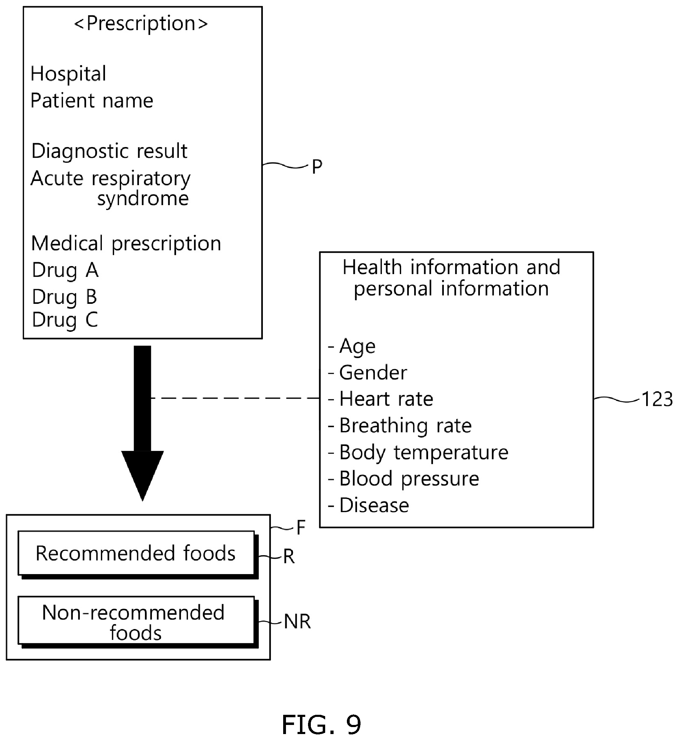

FIG. 9 is a block diagram illustrating an operation in which the electronic device 20 obtains information on recommended foods R suitable for consumption together with the drug components included in the prescription P and non-recommended foods NR unsuitable for consumption together with, or capable of causing side effects when consumed together with, the drug components included in the prescription P, based on the prescription P information, health information and personal information 123 according to an embodiment of the present disclosure.

Referring to FIG. 9, the health information may be information on a user's heart rate, breathing rate, body temperature, blood pressure, brain wave, and so on.

In addition to the above, it also means information related to overall health conditions of a user such as current diseases, body type, allergies, dietary restrictions, and other drugs being taken. For example, there is a need for low-salt diet in patients with hypertension, and restriction on sugar intake in diabetic patients, and food information may vary based on this, but is not limited thereto.

The personal information includes personal characteristics such as gender, age, weight, eating habits, and cultural background of the user. For example, in case of an elderly person, it is necessary to eat soft food due to deglutition (swallowing) dysfunction, or customized food recommendation according to a specific region's eating habits may be possible, but is not limited thereto.

Although the above-described embodiments have been described as being implemented by operations of a system including the user terminal, the server 100 and the affiliated institution terminal 30, they may be implemented only by an operation of the user terminal.

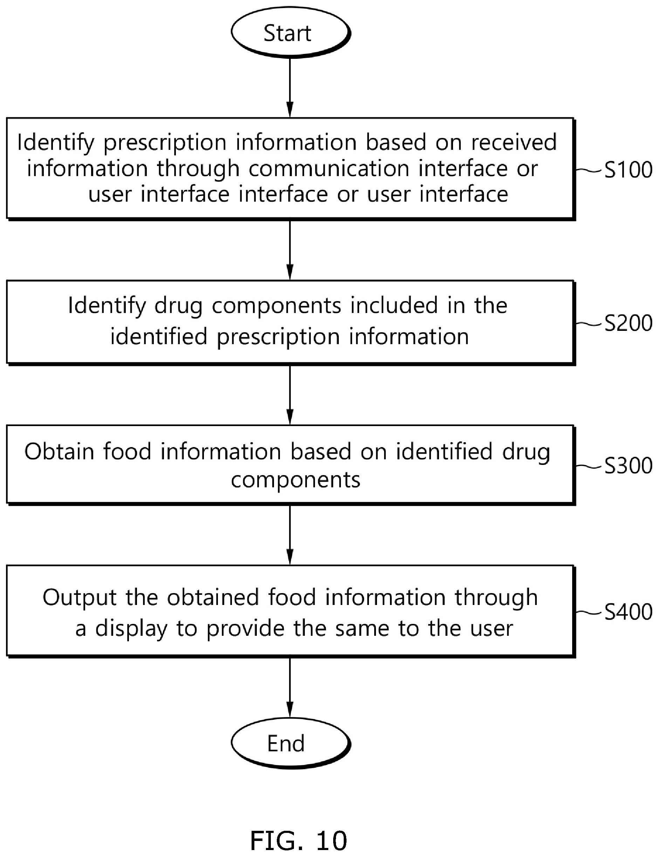

FIG. 10 is a flowchart illustrating an operation of the electronic device 20 according to an embodiment of the present disclosure.

Referring to FIG. 10, the electronic device 20 may identify prescription P information based on the received information through the communication interface 21 or the user interface 22 (S100).

The electronic device 20 may identify the drug components included in the identified prescription P information (S200).

More specifically, the electronic device 20 may identify text for prescription P information based on a user input received through the user interface 22.

At this time, the user terminal may obtain a prescription P image through a camera and identify text included in the obtained prescription P image to identify prescription P information.

The prescription P information includes data such as a patient name, drug name, dosage, and number of doses, and text identification may be generally performed through an optical character recognition (OCR) technology.

Optical character recognition (OCR) may be a technology for automatically recognizing character data such as letters and numbers included in an image or scanned document and converting the recognized characters into digital text.

The basic operation principle of the OCR consists of a process for detecting an area including text in an image and then segmenting individual characters within the corresponding area to determine what each character is. Initially, it was mainly used to recognize standardized printed characters from black-and-white documents input by a scanner, but now it can recognize photographs taken with a smartphone camera, handwritten texts, tables and even signs or license plates in natural images.

The electronic device 20 may perform an image preprocessing operation. This is a step of removing noise from the image and correcting a tilted or dark background to facilitate character recognition.

The electronic device 20 may perform a character recognition operation. In this process, the character patterns are compared and the corresponding character is predicted using an artificial intelligence-based algorithm.

The electronic device 20 may perform a postprocessing operation. Incorrectly recognized characters are corrected or the text is organized into natural text by understanding the context. Recently, a language model is used in the postprocessing process to automatically correct words according to the context.

The OCR technology may be implemented in a deep learning-based OCR method as well as the conventional pattern recognition method. Representative models include convolutional recurrent neural network (CRNN), an attention-based model, and a transformer-based recognition network, which ensure high recognition rate even for complex sentence structures or various fonts. In particular, it is possible to accurately extract text by considering the meaning of sentences in combination with natural language processing (NLP) technology.

At this time, the text may include information on a hospital name, a medical institution name, a patient name, drug prescription information, and disease information of the patient, but is not limited thereto.

The camera is configured to convert visual information into digital images and may be used to collect medical information written on a paper document. For example, the internal camera of a smartphone or a lens included in an OCR scanner may correspond thereto. However, this is not limited thereto.

According to various embodiments, the present disclosure relates to a technology for accurately extracting character information by processing a prescription image, and more specifically, to an electronic device 20 that combines an image preprocessing technique and a character recognition model to ensure a high recognition rate even in environments having low resolution or illumination changes, and includes a multi-language recognition function capable of handling prescription documents composed of multiple languages.

The electronic device 20 receives a prescription image input from a user and performs image preprocessing and a character recognition algorithm to recognize text information in the corresponding image. In particular, the electronic device 20 applies an adaptive binarization technique to maintain recognition accuracy even when the image quality is low or there are distortions, lighting imbalance, etc. This technique dynamically performs pixel binarization according to local brightness changes of the image, thereby improving clarity of letter contours.

The electronic device 20 also applies a noise removal filter developed by itself to remove noise included in an image. This filter can maintain text contours even in low-resolution images and effectively remove background noise, thereby achieving character extraction accuracy of 98% or more. This is measured to be about 15% higher than conventional simple threshold-based processing techniques.

The electronic device 20 uses a convolutional recurrent neural network (CRNN) model for character recognition. The CRNN is a deep learning model that combines a CNN-based visual feature extraction and a RNN-based sequence recognition structure, and can simultaneously learn morphological features of text included in the prescription image and sequential semantics.

The electronic device 20 also includes a multi-language optical character recognition (OCR) engine trained to automatically process prescription documents written in multiple languages. The engine simultaneously supports character systems such as Korean, English, and Japanese, identifies the main language of an input prescription document through automatic language detection, and applies recognition weights optimized for each language, thereby ensuring high universality and recognition rate for prescription documents from various countries.

As a result, the electronic device 20 implements a prescription character recognition system robust to various variables such as distortion, noise, low resolution, and multiple languages that may occur in an actual medical environment, providing superior performance compared to conventional OCR technology and greatly contributing to the digitization and automation of medical data.

The electronic device 20 may identify the prescription P information by identifying text included in the obtained prescription P image.

The prescription P information may include data such as a patient name, drug name, dosage, and number of doses, but is not limited thereto.

The electronic device 20 may obtain the food information F based on the identified drug components (S300).

The electronic device 20 may obtain the food information F corresponding to drug components included in the prescription P based on the outputted value obtained by inputting the prescription P information into the artificial intelligence model 121.

The electronic device 20 may obtain the food information F corresponding to drug components included in the prescription P based on the outputted value obtained by inputting the prescription P information into the artificial intelligence model 121.

The artificial intelligence model 121 is an algorithm system that performs prediction or classification of input values based on data and may be a model used for prediction such as drug interaction or nutrient analysis in the medical and food fields.

The food information F may be data on interaction with the drug component, absorption promotion or inhibition effect, foods to avoid when taking the drug, etc., and the food information F may be information on recommended foods R that can have a positive effect on the body when taken with the drug components and non-recommended foods NR that cause negative effects or side effects to the body when taken with the drug components.

In addition, the food information F may include foods to be avoided in connection with drug administration of a user, ingredients that can increase or decrease the effect of drugs, advice on diet, and so on. For example, there is information indicating that grapefruit may affect the metabolism of some hyperlipidemia drugs, and isoniazid should be taken with restriction on high-fat foods, which may be used for personalized medication guidance, but is not limited thereto.

For example, there are interactions between warfarin and green vegetables containing a large amount of vitamin K, or calcium supplements and dairy products, which are used to provide medication guidance according to the influence of specific foods on drug efficacy.

The electronic device 20 may obtain food information F corresponding to the identified drug components based on the drug-food interaction database 122.

The drug-food interaction database 122 is a set of information that stores the relationship between specific drugs and specific foods in an organized manner, including food-related data that affect drug absorption rate, metabolism, efficacy, or side effects.

For example, there are Micromedex's Drug-Food Interactions and the FDA's Drug Interaction Database, which can be used to automatically extract contraindicated foods or recommended foods for each drug component, but are not limited thereto.

According to various embodiments of the present disclosure, a technology for constructing a database including information on interactions between drugs and foods, and providing customized food information to users based on the constructed database is provided. More specifically, an electronic device 20 that generates and utilizes a dynamic database including food items reflecting regional characteristics such as Korean food culture, and continuously updated based on user feedback is provided.

The electronic device 20 builds its own database to provide information on the interaction between drugs and foods, wherein the corresponding database is generated by combining publicly disclosed global interaction data with user feedback data. At this time, the public data refers to commonly used drug interaction materials such as Micromedex and DrugBank, and the user feedback data refers to responses actually experienced by a user after taking a drug, inconveniences, preferred food information, and the like.

The electronic device 20 includes information reflecting regional specificity such as Korean food culture in the database. For example, drug interaction information on traditional foods such as kimchi, soybean paste, fermented soybean paste, and water parsley is recorded, which is not reflected in existing global databases. The electronic device 20 may provide more realistic and culturally relevant information by adding items for these regional foods.

The electronic device 20 periodically updates data items in the database based on user feedback to maintain the accuracy and currency of the database. Specifically, by analyzing medication history, diet diary, adverse reaction records, and the like collected from a user, new interaction information is inferred, and verified contents are added to or modified in the database. The update may be automatically performed every month or when a specific trigger occurs.

In this manner, the database constructed and utilized by the electronic device 20 may be a hybrid interaction information set based on global standard information but including local unique foods, which can be used as base data for providing user-customized information.

In addition, the electronic device 20 may store a database update history, an update period, and user-specific reflection records in a log form to secure data reliability and transparency.

As a result, the present invention can supplement limitations of global interaction data, provide customized food information by considering different eating habits according to culture, and realize an evolutionary database system in which actual user experience is reflected.

The electronic device 20 may obtain food information F including recommended food information that a user is advised to consume with a drug and non-recommended food information that may cause side effects when consumed by the user with the drug based on the identified drug components.

The identified drug component is an active ingredient of the medicine specified in the prescription P and is a main reference for affecting how the corresponding component acts in the body, metabolic pathways, excretion characteristics, etc. For example, drug components such as 'acetaminophen' and 'warfarin' may be included, and interactions with foods may be analyzed based thereon, but is not limited thereto.

The recommended food information refers to a food that is good to take together with a drug in order to increase the effect of the drug or prevent side effects such as gastrointestinal disorders. For example, there are orange juice which helps absorption when taking an iron supplement, and soft high-protein diet which aids digestion when taking a digestive enzyme agent, but are not limited thereto.

The non-recommended food information is a food that may interfere with drug metabolism or cause side effects, for example, spinach rich in vitamin K when warfarin is taken, and the interaction between grapefruit and some hyperlipidemia treatment drugs. However, this is not limited thereto.

According to various embodiments of the present disclosure, when the ratio of antibiotics among the drug components included in the prescription P is equal to or greater than a predetermined first value, the electronic device 20 may identify, as recommended food R information F, foods such as yogurt, kimchi, soybean paste, fermented soybean paste, which contain a predetermined amount or more of probiotics; foods such as water parsley, broccoli, or foods containing vitamin A, vitamin C, vitamin E, etc., which are capable of protecting liver function; or health functional foods including probiotics or ingredients capable of protecting liver function.

In addition, the electronic device 20 may identify foods containing high fat, high sugar, alcohol and caffeine as non-recommended food NR information F when the ratio of antibiotics among the drug components included in the prescription P is equal to or greater than the first predetermined value.

The electronic device 20 may identify food including a large amount of probiotics and having a moisture content equal to or greater than a certain level, among foods including a large amount of probiotics, such as stews and soups including kimchi soup, kimchi stew, soybean paste soup, soybean paste stew, fermented soybean paste stew, water parsley soup, and broccoli soup, which include kimchi, soybean paste, fermented soybean paste, water parsley, and broccoli, as recommended food R information F when the ratio of antibiotics among the drug components included in the prescription P is equal to or greater than the second predetermined value (which is larger than the first predetermined value).

That is, according to the above-described embodiment, since antibiotics may kill beneficial bacteria in the intestine and cause indigestion, it is possible to provide recommended food R information F that enables sufficient intake of beneficial bacteria in the intestine and improves liver function, and when the ratio of antibiotics is considerably high, stews and soups that provide beneficial bacteria and also contain a large amount of moisture may be identified as recommended food R information F.

In addition, the electronic device 20 may identify, as recommended food information F, ingredients including cabbage, potato, sweet potato, banana which can protect the stomach; and ingredients including broccoli, water parsley, garlic, onion, or ingredients containing more vitamin C and E than a predetermined amount when the ratio of an analgesic among the drug components included in the prescription P is equal to or greater than a third predetermined value.

The electronic device 20 may identify foods containing caffeine, alcohol, and fat in a predetermined amount or more of fat, processed foods, and instant foods as non-recommended food NR information R when the ratio of an analgesic among the drug components included in the prescription P is equal to or greater than a third predetermined value.

That is, according to the above-described embodiment, since an analgesic may cause a burden on the stomach, liver, and kidney, recommended food R information F capable of improving functions of the stomach, liver, and kidney may be provided.

The electronic device 20 may obtain food information F including recommended food information that a user is advised to consume with a drug and non-recommended food information that may cause side effects when consumed by the user with the drug based on the user's health information and personal information 123.

The health information may be information on a user's heart rate, breathing rate, body temperature, blood pressure, brain wave, etc.

In addition to the above, it also means information related to overall health conditions of a user such as current diseases, body type, allergies, dietary restrictions, and other drugs being taken. For example, there is a need for low-salt diet in patients with hypertension, and restriction on sugar intake in diabetic patients, and food information may vary based on this, but is not limited thereto.

The personal information includes personal characteristics such as gender, age, weight, eating habits, and cultural background of the user. For example, in case of an elderly person, it is necessary to eat soft food due to deglutition (swallowing) dysfunction, or customized food recommendation according to a specific region's eating habits may be possible, but is not limited thereto.

According to various embodiments of the present disclosure, as described above, the electronic device 20 may perform an OCR on the prescription P image to identify text included in the prescription P and identify drug components corresponding to the identified text or identify the drug components included in the prescription P based on user input through the user interface 22.

The electronic device 20 may input the identified drug components into a food recommendation model and obtain food information F including recommended foods R that give positive effects to health and nutrition of the body when consumed with the drug components and non-recommended foods NR that may cause side effects when consumed with the drug components based on an outputted value of the food recommendation model. Herein, the food information F may be information about food, beverages, ingredients, or health functional foods, but is not limited thereto.

In addition to the above, the electronic device 20 may obtain food information F including recommended foods R that give positive effects to the body in terms of health and nutrition when taken together with the identified drug components and non-recommended foods NR that may cause side effects when taken together with the drug components by referring to the drug-food interaction database 122.

In addition, the electronic device 20 may identify the number of different drug components included in the prescription P. For example, the electronic device 20 may identify drug components corresponding to an analgesic and drug components corresponding to an antibiotic as different drug components, thereby identifying that the number of drug components is two.

The electronic device 20 may identify the number of drug components prescribed by the user through the prescription P at predetermined intervals.

When the number of drug components is equal to or greater than a threshold value and the age of the user is less than a threshold age, the electronic device 20 may obtain food information F including a number of non-recommended foods NR that is equal to or greater than a first threshold multiple times the number of recommended foods R. At this time, the number of foods may mean the number of food items or the number of food types.

When the number of drug components is equal to or greater than a threshold value and the age of the user is equal to or greater than a threshold age, the electronic device 20 may obtain food information F including a number of non-recommended foods NR that is equal to or greater than a second threshold multiple (a value larger than the first threshold multiple) times the number of recommended foods R.

When the number of drug components is less than a threshold value, the electronic device 20 may obtain food information F including more recommended foods R than non-recommended foods NR.

In addition, the electronic device 20 may identify the current age of the user at each predetermined period of the user, and identify may a second threshold multiple when the user's current age is greater than the above-described threshold age and in proportion to the difference between the current age of the user and the threshold age.

That is, as the number of components of prescribed drugs increases, the physical condition is poorer and more diseases are likely present, and thus food information F on foods requiring caution with respect to side effects may be preferentially provided over recommended foods, and in particular, since health may be more vulnerable in the case of an elderly user, a greater amount of food information F on foods requiring caution may be provided so as to be more helpful for promoting the user's health.

In addition, as the user ages, the value of the second threshold multiple is adjusted to a larger value in proportion thereto so that information on non-recommended foods to be cautious of when the user is elderly can be provided.

According to the above-described embodiment, when the disease of a user is alleviated and the number of prescribed drug components decreases, information on recommended foods R may naturally be provided more than information on non-recommended foods NR, thereby also promoting the user's general health.

The electronic device 20 may output the obtained food information F through a display 23 to provide the same to the user (S400).

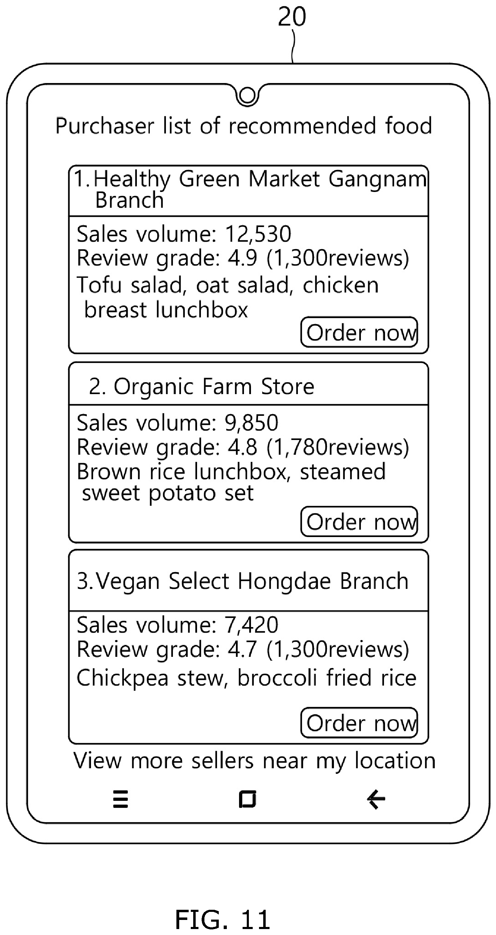

FIG. 11 is a diagram illustrating a screen on which the electronic device 20 outputs, through a display 23, a seller list M selling recommended foods for drug components included in prescription information to provide the same to the user according to an embodiment of the present disclosure.

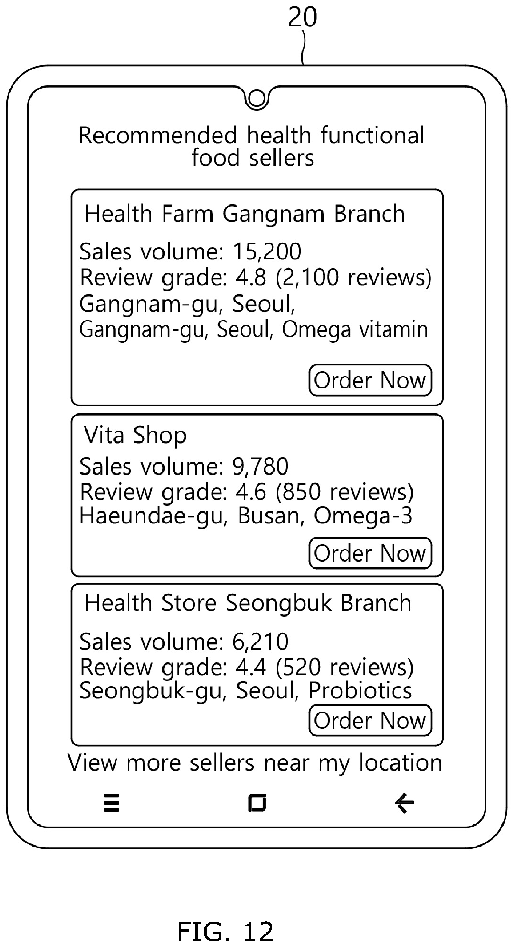

FIG. 12 is a diagram illustrating a screen on which the electronic device 20 outputs, through a display 23, a seller list M selling recommended health functional foods for the drug components included in the prescription information to provide the same to the user according to an embodiment of the present disclosure.

Referring to FIGS. 11 and 12, when a user input for selecting at least one of the foods displayed on the display 23 is received through the user interface 22 while the food information F is output through the display 23, the electronic device 20 may output sales information about the selected food through the display 23 to provide the same to the user.

The sales information may include data such as a price of the selected food, a seller, delivery options, and a purchase link.

For example, the sales information may include a name of a seller selling food, sales volumes, review grade (review score), types or names of foods or ingredients.

In addition, the electronic device 20 may output an order button and a link to connect to a seller near the location of a user together to provide the same to the user.

The electronic device 20 may output a plurality of seller lists M selling the selected foods through the display 23 to provide the same to the user.

The plurality of seller lists M refers to a list in which information on several stores or platforms providing the same product is listed and may include items such as 'Healthy Green Market Gangnam Branch', 'Organic Farm Store', and 'Vegan Select Hongdae Branch', as shown in FIG. 11. However, this is not limited thereto.

The electronic device 20 may provide the display order of a plurality of sellers in an sorted form based on the evaluation scores and the sales volumes.

The evaluation score is a quantified satisfaction value or score input through user reviews and the like, and the sales volume is an index indicating how much of the corresponding product has been sold for a certain period of time. For example, this may be a method in which a seller with 'review score of 4.9 points & monthly sales of 1,000 units' is positioned at the top, and a seller with 'review score of 3.5 points & monthly sales of 50 units' is positioned at the bottom, but is not limited thereto.

The electronic device 20 may output, through the display 23, a list with a seller display order sorted such that the sellers to display a list in which the sellers having high evaluation scores and high sales volumes are positioned at the top of the list and the sellers having low evaluation scores and low sales volumes are positioned at the bottom of the list to provide the same to the user.

Such a sorting method is a part of the UX design for providing intuitive criteria to users' purchase decisions, and may include 'sorting by best-selling products', 'listing from most reputable sellers', etc., but is not limited thereto.

In another embodiment, the electronic device 20 may learn a previous purchase history and a preferred product category of a user, and sort the seller list M based on evaluation score and sales volume data of a user group of sellers having purchase patterns similar to those of the user. Accordingly, it is possible to provide a customized list optimized for individual users' tastes and purchasing habits, thereby increasing user satisfaction and purchase efficiency.

The electronic device 20 may periodically update the evaluation score and sales volume data of each seller displayed in the seller list M to reflect the current trend and real-time popularity change of a seller on the list order. As such, the user can be provided with an environment where he/she can easily identify and select the most trendy and popular seller based on the latest information.

The electronic device 20 may collect evaluation scores and sales volumes for each of the plurality of sellers, and update the seller list M based on the collected evaluation score and sales volume information for each of the plurality of sellers at a predetermined period.

At this time, the predetermined period refers to a time interval at which the system automatically performs repetition, and may be updated once per day or every hour, but is not limited thereto.

The list update refers to a process of resorting or applying changed contents by reflecting new information in the existing seller list. For example, this may include dynamic changes such as adding a new seller or moving a seller with increased sales volumes to the top of the list, but is not limited thereto.

The electronic device 20 may obtain information on a seller selected by the user for purchase.

The information on the selected seller may mean an identifier of a seller clicked by the user to purchase a specific product or added to a shopping cart, location information, and a name of the seller. For example, it may be a store name or a shopping mall URL of an online shopping mall selling a specific health functional food. However, this is not limited thereto.

The electronic device 20 may obtain the purchase history database 125 of the user based on the obtained seller information.

The purchase history database 125 refers to data that stores records related to products and sellers selected or purchased by the user in the past in an organized manner. For example, there may be purchase histories of health foods, lists of foods consumed together with drugs, but is not limited thereto.

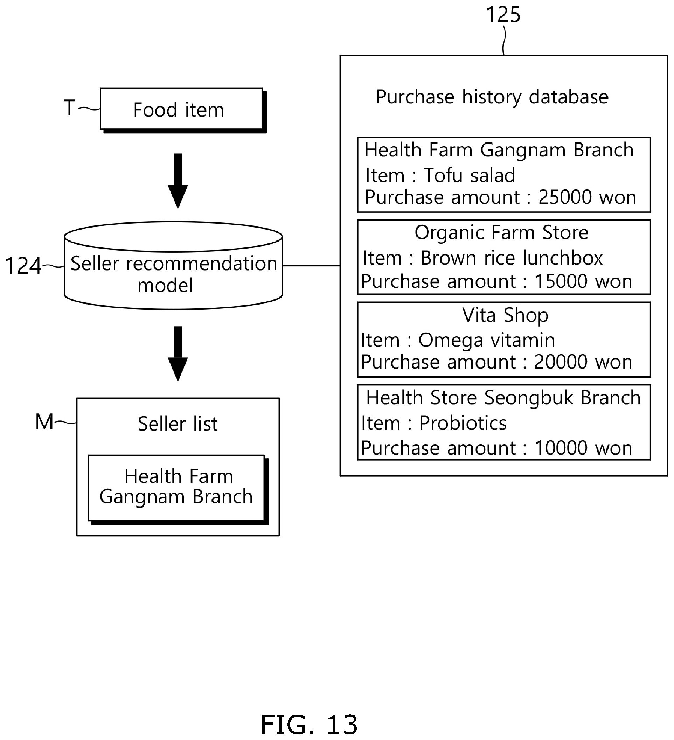

FIG. 13 is a diagram illustrating an operation in which the electronic device 20 inputs a food item T of a user into a seller recommendation model 124 learned based on a user purchase history to obtain a seller list M corresponding to the food item T according to an embodiment of the present disclosure.

Referring to FIG. 13, the electronic device 20 may learn a seller recommendation model 124 based on purchase history database 125.

Specifically, the electronic device 20 may learn a seller recommendation model 124 based on the food item T included in the purchase history and information about a seller selling the corresponding food item T.

The seller recommendation model 124 may be an artificial intelligence based algorithm, the artificial intelligence model 121, or a deep learning model that predicts a seller likely to be preferred in future by analyzing the past purchase patterns of the users. For example, there may be a collaborative filtering model or a content-based filtering model, but is not limited thereto.

The electronic device 20 may identify the food item T selected by a user through the user interface 22 and input the food item T into the seller recommendation model 124 to obtain a recommended seller list M based on an outputted value.

When a user input for selecting at least one food from the recommended foods R information F is received through the user interface 22, the electronic device 20 may identify the selected food included in the recommended foods R information F.

The electronic device 20 may identify a recommended seller corresponding to the selected food based on an outputted value obtained by inputting the identified selected food into the seller recommendation model 124.

The electronic device 20 may generate a seller list M including the identified recommended sellers.

According to various embodiments of the present disclosure, the present invention relates to a technology for recommending an optimal seller based on purchase history, preference, regional information, etc., of a user. More specifically, an electronic device 20 capable of providing highly accurate customized seller information to a user by applying a hybrid recommendation model that combines collaborative filtering and content-based filtering is provided.

The electronic device 20 collects the past purchase history data of the users and performs content-based filtering by analyzing attribute information of a product related to the corresponding history. Content-based filtering is a method for recommending an item having similar attributes to items that the user has previously preferred or purchased, and may provide recommendation results optimized for individual tastes of the user.

At the same time, the electronic device 20 performs collaborative filtering by cross-analyzing evaluation and behavior data of a plurality of users. Collaborative filtering is a method for predicting an item that has not yet been accessed by a specific user based on behaviors of a group of users having similar preferences, which can be usefully utilized even when personal data are insufficient.

The electronic device 20 applies a hybrid recommendation model that combines the two filtering techniques. The hybrid model can simultaneously secure accuracy and diversity of recommendations by combining advantages of collaborative filtering and content-based filtering. At this time, the recommendation result is calculated in a weighted sum manner of the results of the two filtering methods or may be adjusted to have different priorities according to situations.

The electronic device 20 also considers user location information to increase the accuracy of recommendation. Based on the current location of the user, distance information to a seller to be recommended is calculated, and, for example, an additional weight is assigned to sellers located within a radius of 5 km. As such, it is possible to recommend sellers that are practically accessible to the user, and the accuracy of recommendation may be increased by about 30% or more compared to the conventional method.

As a result, the electronic device 20 may improve the quality of personalized recommendations for each user by using a combined structure of collaborative filtering and content-based filtering, and applying regional weights thereto. This may contribute to improving user experience and increasing purchase conversion rate in an integrated online/offline distribution environment.

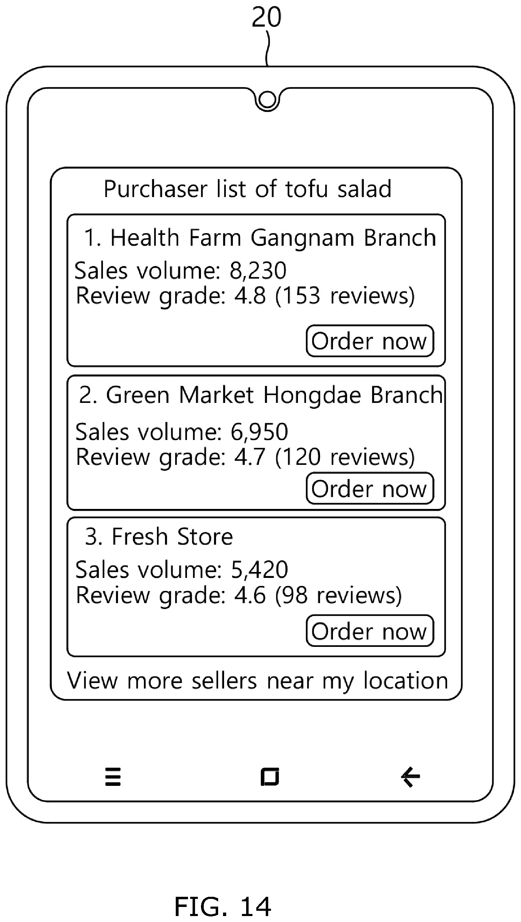

FIG. 14 is a diagram illustrating a screen on which the electronic device 20 outputs a recommended seller list M through the display 23 to provide the same to the user according to an embodiment of the present disclosure.

Referring to FIG. 14, the electronic device 20 may output the generated seller list M through the display 23 to provide the same to the user.

The seller list may be such that, based on the user's purchase history, during a predetermined period, sellers having a higher number of purchases or a higher amount spent on purchases by the user are arranged at the top, and sellers having a lower number of purchases or a lower amount spent on purchases by the user are arranged at the bottom, but is not limited thereto.

According to various embodiments of the present disclosure, the present disclosure relates to a technology for integrating public data, private data and user data in a welfare card system and optimizing provision of welfare services based on the integrated data. In particular, the present disclosure relates to an electronic device 20 that clarifies a data flow structure of a welfare card system and provides customized welfare benefits to a user through data interconnection with various affiliated institutions.

The electronic device 20 analyzes and processes public welfare information, private medical/pharmacy information, and individual health data of the user by linking them together. The public data includes welfare qualification information and benefit payment history provided from the National Health Insurance Service, the Bokjiro (Korea Welfare Portal), and a social security information system. Private data may be collected from internal systems of medical institutions and pharmacies such as prescription histories of pharmacies, treatment records of hospitals, and results of health checkups. User data includes health-related information (e.g., heart rate and activity level) collected through mobile terminals or wearable devices.

The electronic device 20 configures an application programming interface (API) for integrating the data, and the corresponding API is designed to process a data flow between public organizations, private organizations, and user terminals in a standardized manner. Specifically, after performing an authentication procedure on each data source, the data are converted into structured data based on JSON or XML, and then merged into an internal integrated storage.

The electronic device 20 applies the HL7 FHIR (Fast Healthcare Interoperability Resources) protocol, which is an international standard for data interconnection with a medical institution or welfare organization. The FHIR is a structure for transmitting and receiving medical data in a standardized form in a digital environment, and the present disclosure performs real-time interconnection with hospitals, pharmacies, welfare organizations, etc., through this standard. For example, medical records provided by a hospital are structured through Observation, MedicationStatement, DiagnosticReport resources of FHIR and connected to a welfare card system.

The electronic device 20 analyzes the linked information in a comprehensive manner to provide customized welfare benefits to users. For example, eligibility for a medication support service is determined based on prescription data of pharmacies, welfare eligibility requirements are evaluated according to public data, and candidates for exercise assistance products or rehabilitation services are selected based on health data collected from wearable devices.

As a result, the electronic device 20 may provide highly advanced welfare customized services in real time through integrated processing of public, private, and user data and inter-institutional data interconnection based on the HL7 FHIR standard, and can effectively resolve inefficiencies and discontinuity problems of existing welfare service systems.

Meanwhile, the various embodiments described above may be implemented by combining two or more embodiments without conflicting with each other.

Specifically, each of a plurality of operations, steps and configurations for implementing one embodiment may be implemented by another embodiment or an operation or step of another embodiment may follow the last operation or step of one embodiment, but is not limited thereto.

Meanwhile, the computer instructions or the computer program for performing the processing operations of various embodiments described above may be stored in a non-transitory computer-readable medium. When the computer instructions or the computer program stored in the non-transitory computer-readable medium are executed by a processor of a specific device, the specific device performs the processing operation according to various embodiments described above.

The non-transitory computer-readable medium refers to a medium that stores data semi-permanently and is readable by a device, rather than a medium that stores data only for a short time such as registers, caches, memories, etc. Specific examples of the non-transitory computer-readable medium may include CDs, DVDs, hard disks, Blu-ray discs, USBs, memory cards, ROMs, etc.

According to an embodiment, the method according to various embodiments disclosed in this document may be included and provided in a computer program product. The computer program product may be traded between a seller and a buyer as a commodity. The computer program product may be distributed in the form of a device-readable storage medium (e.g., compact disc read only memory (CD-ROM)), or may be distributed online (e.g., by downloading or uploading) through an application store (e.g., Play StoreTM) or directly between two user devices (e.g., smartphones). In the case of online distribution, at least part of the computer program product (e.g., downloadable app) may be temporarily generated or stored in a machine-readable storage medium such as a memory of a manufacturer's server, an application store server, or a relay server.

Although the preferred embodiments of the present disclosure have been illustrated and described above, the present disclosure is not limited to the above-described specific embodiments, and various modifications can be made by those skilled in the art to which the present disclosure pertains without departing from the scope of the present disclosure as claimed in the claims, and such modifications should not be understood separately from the technical spirit or prospect of the present disclosure.

## Claims

1. An electronic device for providing a customized service on drug prescription in connection with an AI green welfare card system based on collection and provision of user data, the electronic device comprising:
a user interface configured to receive a user input;
a communication interface configured to perform communication connection with a server;
a display configured to output visual content; and
a processor connected to the user interface, the communication interface and the display, and performing control operations by executing at least one instruction,
wherein the processor identifies prescription information based on information received through the communication interface or the user interface, identifies drug components included in the identified prescription information, obtains food information based on the identified drug component, and outputs the obtained food information through the display to provide the same to the user.

2. The electronic device of claim 1, wherein the processor:
obtains a prescription image through a camera;
preprocesses the obtained prescription image; and
identifies text included in the obtained prescription image to identify prescription information using an OCR technology.

3. The electronic device of claim 1, wherein the processor inputs the prescription information into an artificial intelligence model to obtain food information corresponding to drug components included in the prescription information based on an outputted value, and
wherein the artificial intelligence model has been trained using the prescription information and the drug components as training data.

4. The electronic device of claim 1, wherein the processor obtains food information corresponding to the identified drug components based on a drug-food interaction database.

5. The electronic device of claim 1, wherein the processor obtains food information including recommended food information to be consumed by a user with a drug and non-recommended food information that may cause side effects when consumed by the user with the drug based on the identified drug components.

6. The electronic device of claim 1, wherein, when a user input for selecting at least one of the foods displayed on the display is received through the user interface while the food information is outputted through the display, the processor outputs sales information of the selected foods through the display to provide the same to the user.

7. The electronic device of claim 1, wherein the processor:
obtains information on a seller selected by the user for purchase,
obtains a purchase history database of the user based on the obtained information on the seller, and
trains a seller recommendation model based on the purchase history database.

8. The electronic device of claim 7, wherein, the processor:
identifies the selected food included in the recommended food information when a user input for selecting at least one food from the recommended food information is received through the user interface;
inputs the identified selected food into the seller recommendation model to identify a recommended seller corresponding to the selected foods based on an outputted value;
generates a seller list including the identified recommended seller; and
outputs the generated seller list through the display to provide the same to the user.

9. An operation method of a system for providing customized services on drug prescriptions in connection with an AI green welfare card system based on collection and provision of user data, the method comprising the steps of:
identifying prescription information by the server based on information received from an external device or a user terminal;
identifying drug components included in the identified prescription information by the server; obtaining food information based on the identified drug components by the server;
transmitting the obtained food information to the user terminal by the server; and
outputting, by the user terminal, the food information received from the server to provide the same to the user.

10. A control method of a system for providing customized services on drug prescriptions in connection with an AI green welfare card system based on collection and provision of user data, the method comprising the steps of:
obtaining, by a user terminal, prescription information through a camera; identifying, by the user terminal, text from a prescription image using adaptive binarization and CRNN-based OCR technology to extract prescription information;
transmitting, by a user terminal, the prescription information to a server;
identifying, by the server, drug components included in the prescription information; obtaining, by the server, recommended foods and non-recommended foods information corresponding to the drug components through a drug-food interaction database including Korean food data and a Transformer-based AI model;
generating, by the server, a seller list of the recommended foods through a hybrid recommendation model trained with user purchase history;
transmitting, by the server, the food information and the seller list to the user terminal; and
outputting, by the user terminal, the food information and the seller list through a display.
